# EUROPÄISCHE PATENTSCHRIFT

(11) **EP 4 054 498 B1**
(45) Veröffentlichungstag und Bekanntmachung des Hinweises auf die Patenterteilung: **25.03.2026**
(21) Anmeldenummer: 20803497.5
(22) Anmeldetag: 05.11.2020
(51) Int. Cl.: A61F 13/20, A61M 31/00

(54) **TAMPON FÜR MEDIZINISCHE UND HYGIENISCHE ZWECKE SOWIE VERFAHREN ZU SEINER HERSTELLUNG**
TAMPON FOR MEDICAL AND HYGIENIC PURPOSES, AND METHOD FOR MANUFACTURING SAME
TAMPON À USAGE MÉDICAL ET HYGIÉNIQUE ET SON PROCÉDÉ DE FABRICATION

(30) Priorität: 08.11.2019 DE 102019217288
(43) Veröffentlichungstag der Anmeldung: 14.09.2022
(73) Patentinhaber: Rennig, Alexander Fabian, 80803 München (DE)
(72) Erfinder: Rennig, Alexander Fabian, 80803 München (DE)
(74) Vertreter: Hoffmann Eitle
(86) Internationale Anmeldenummer: PCT/EP2020/081103
(87) Internationale Veröffentlichungsnummer: WO 2021/089686

(56) Entgegenhaltungen:
- EP-A1- 0 415 087
- EP-A1- 0 415 087
- EP-A1- 2 949 315
- EP-B1- 0 415 087
- US-A1- 2004 043 071
- US-A1- 2004 043 071
- US-A1- 2020 323 699
- MAHDAWI ARWA: "Are "weed tampons" the answer to period pain?", THE GUARDIAN, 28 May 2019 (2019-05-28), XP055774877, Retrieved from the Internet <URL:https://www.theguardian.com/commentisfree/2019/may/28/are-weed-tampons-answer-to-period-pain-cbd-suppositories-pms> [retrieved on 20210211]
- MAHDAWI ARWA: "Are "weed tampons" the answer to period pain?", THE GUARDIAN, 28 May 2019 (2019-05-28), XP055774877, Retrieved from the Internet <URL:https://www.theguardian.com/commentisfree/2019/may/28/are-weed-tampons-answer-to-period-pain-cbd-suppositories-pms> [retrieved on 20210211]

## Beschreibung

### Gebiet der Erfindung

Die Erfindung betrifft einen Tampon zur vaginalen Anwendung für medizinische und hygienische Zwecke sowie ein Verfahren zu dessen Herstellung.

### Hintergrund der Erfindung

Tampons für hygienische Anwendungen bestehen in der Regel aus einem Gemisch aus Baumwolle und Zellwolle, dem gegebenenfalls Quellstoffe zur Erhöhung des Flüssigkeitsabsorptionsvermögens hinzugefügt werden.

In gleicher Weise sind auch medizinische Suppositorien zur vaginalen Anwendung bekannt, deren Funktion aber nicht darin besteht, Körperflüssigkeiten aufzusaugen, sondern Wirkstoffe abzugeben. Dabei macht man sich den Umstand zu Nutze, dass über die Vaginalschleimhaut Wirkstoffe sehr effektiv und direkt dem Blutkreislauf zugeführt werden können.

Aus der EP 0 415 087 A1 ist ein Tampon für medizinische oder hygienische Zwecke bekannt, der einen Tamponmantel aus gehärtetem Collagen- oder Gelatineschaum umfasst, der mit Wirkstoffen imprägniert ist.

In einem Artikel in The Guardian vom 28. Mai 2019 unter dem Titel "Are "weed tampons" the answer to period pain?" berichtet die Autorin Arwa Mahdawi über Suppositorien, die Cannabidiol umfassen.

Die US 2004/043071 A1 stellt den nächstkommenden Stand der Technik dar.

### Darstellung der Erfindung

Regelbeschwerden sind häufig mit Schmerzen und krampfartigen Zuständen verbunden. Daher besteht die Aufgabe der Erfindung darin, ein Produkt zu entwickeln, das eine Kombination aus einem medizinischen Suppositorium und einem Tampon darstellt, indem es einerseits Körperflüssigkeiten aufsaugt und andererseits Regelbeschwerden lindert.

Diese Aufgabe wird durch einen Tampon zur vaginalen Anwendung für medizinische und hygienische Zwecke mit den Merkmalen des Anspruchs 1 sowie durch ein Verfahren zur Herstellung eines derartigen Tampons mit den Merkmalen des Anspruchs 6 gelöst. Bevorzugte Ausführungsformen folgen aus den übrigen Ansprüchen.

Der erfindungsgemäße Tampon zur vaginalen Anwendung für medizinische und hygienische Zwecke mit einem Einführende, einem Mittelabschnitt und einem Rückholende umfasst einen Tamponkern aus einem Flüssigkeit absorbierenden, gepressten Faserverbundstoff, eine Rückholeinrichtung, vorzugsweise einen Rückholfaden, die sich vom Rückholende über dieses hinaus erstreckt, vorzugsweise eine bereichsweise auf dem Tamponkern aufgebrachte, äußere Gleitmittelbeschichtung, sowie eine Wirkstoffsubstanz zumindest im Bereich des Einführendes, welche im Bereich des Einführendes als Beschichtung oder als stückiges Material vorliegt, das mit dem Tamponkern verbunden ist. Die Wirkstoffsubstanz umfasst ein hydrogeniertes Fett als Trägersubstanz, und eine aktive Substanz umfassend mindestens eine chemische Substanz aus der Gruppe der Cannabinoide, insbesondere Cannabidiol.

Der erfindungsgemäße Tampon erfüllt somit gleichzeitig zwei Aufgaben, nämlich die übliche Flüssigkeit absorbierende Wirkung wie auch nach dem Einführen die Abgabe der Wirkstoffsubstanz mit den darin enthaltenen Cannabinoiden, die eine schmerzlindernde und entkrampfende Wirkung entfaltet. Die Wirkstoffsubstanz muss dabei so hergestellt werden, dass diese erst bei Körpertemperatur schmilzt und vom Körper aufgenommen werden kann. Der Schmelzpunkt der Wirkstoffsubstanz wird dabei unter anderem über die Auswahl des hydrogenierten Fetts als Trägersubstanz eingestellt.

Die Menge der aktiven Substanz pro Tampon liegt zwischen 25 und 300 mg und bevorzugt zwischen 50 mg und 300 mg. Die chemische Substanz ist dabei im Wesentlichen Cannabidiol (CBD). Cannabidiol ist dabei der wichtigste Vertreter aus der Gruppe der Cannabinoide, von denen in der Cannabispflanze zumindest 113 wissenschaftlich untersuchte chemische Verbindungen aus der Gruppe der Cannabinoide enthalten sind. Der Anteil an Tetrahydrocannabinol (THC) richtet sich danach, ob und in welchem Umfang eine psychoaktive Wirkung erwünscht ist. Sollte diese nicht erwünscht sein, sollte THC allenfalls in Spuren in der Wirkstoffsubstanz enthalten sein. THC ist die in der Cannabispflanze enthaltene, psychoaktive Substanz, welcher der Hauptanteil der psychoaktiven Wirkung zugesprochen wird.

Nach einer bevorzugten Ausführungsform der Erfindung ist der Faserverbundstoff organischer Baumwollfaserstoff.

Baumwollfaserstoff hat gegenüber dem im Stand der Technik meist eingesetzten synthetischen Gewebe den Vorteil, dass es sich um ein Naturprodukt handelt. Bei der vaginalen Einführung und Verwendung von Tampons kommt es immer in einem geringen Umfang zu einem Faserverlust, weshalb handelsübliche Tampons in der Regel mit einer Beschichtung, häufig aus gehärtetem Collagenschaum oder Gelatineschaum mit pflegenden Wirkstoffen versehen sind, welche dem Faserverlust entgegenwirken soll. Da sich ein Faserverlust nicht vollständig vermeiden lässt, ist es vorteilhaft als Faserverbundstoff organischen Baumwollfaserstoff einzusetzen. Der Begriff "organischer Baumwollfaserverbundstoff" bezeichnet dabei genetisch nicht modifizierte Baumwolle aus organischem Anbau, die nicht mit synthetischen landwirtschaftlichen Chemikalien wie Pestiziden und Düngemitteln behandelt wurde.

Vorzugsweise liegt eine den pH-Wert beeinflussende Substanz in einem Volumenanteil von etwa 2 % in der Wirkstoffsubstanz vor und ist weiter bevorzugt eine pH-Puffersubstanz, die Milchsäure umfasst.

Im pharmazeutischen Bereich wird Milchsäure als pH-Puffersubstanz eingesetzt. Die Milchsäure hat allerdings den zusätzlichen Vorteil, dass es bereits bekannt ist, Milchsäure in Suppositorien zur Stärkung des vaginalen Milieus einzusetzen. Die Verwendung von Milchsäure besitzt somit die Doppelfunktion, die Wirkstoffsubstanz zu stabilisieren und gleichzeitig bei der Anwendung des Tampons pflegend und regulierend zu wirken.

Nach einer nicht erfindungsgemäßen Ausführungsform der Erfindung umfasst die aktive Substanz umfassend eine chemische Substanz aus der Gruppe der Cannabinoide überwiegend Cannabidiol, das in Öl als Trägersubstanz enthalten ist. Bevorzugt werden Hanfsamenöl, Kokosöl und Olivenöl eingesetzt. Dabei ist Hanfsamenöl besonders bevorzugt, da es reich an Omega 3- sowie Omega 6-Fettsäuren ist. Unter "überwiegend" ist hier zu verstehen, dass in der aktiven Substanz Cannabidiol in einem Volumenanteil von mindestens 50% des Gesamtvolumens der in der aktiven Substanz enthaltenen Cannabinoide enthalten ist.

Nach einer alternativen, nicht erfindungsgemäßen Ausführungsform der Erfindung umfasst die aktive Substanz umfassend eine chemische Substanz aus der Gruppe der Cannabinoide überwiegend Cannabidiol, das in einer Paste enthalten ist, deren Schmelzpunkt höher als 25 °C ist und bevorzugt höher als 27°C ist. Derartige Pasten sind bereits auf dem Markt erhältlich, so dass der Prozess der Extraktion oder des Kaltpressens oder aber die Gewinnung in einem Bioreaktor ausgehend von Cannabispflanzen und insbesondere der Pflanze Cannabis Sativa L mit einem hohen natürlichen Anteil an Cannabidiol nicht mehr beschrieben werden muss.

Da gewünscht ist, dass die aktive Substanz in möglichst reiner Form angewendet wird, umfasst erfindungsgemäß die aktive Substanz zu mindestens 90 Vol.-% Cannabidiol, das als Isolat oder Destillat vorliegt. Weiterhin beträgt der Volumenanteil der aktiven Substanz in der Wirkstoffsubstanz zwischen 20% und 80%.

Bei der Herstellung eines Destillats wird die gewünschte aktive Substanz bereits möglichst hoch angereichert, während nach der Herstellung eines Isolats nur noch die gewünschte chemische Substanz und allenfalls Spuren anderer Substanzen in der aktiven Substanz vorhanden sind. Isolate und Destillate von Cannabidiol sind im Handel in kristalliner Form oder in fester, gepresster Form erhältlich.

Abhängig davon, in welcher Form die aktive Substanz in der Wirkstoffsubstanz enthalten ist, liegt ein geringer oder aber beträchtlicher Volumenanteil der aktiven Substanz in der Wirkstoffsubstanz vor. Auf diese Weise wird die Einzeldosis an Cannabinoiden und insbesondere Cannabidiol pro Tampon auf einen gewünschten Zielwert eingestellt.

Nach einer bevorzugten Ausführungsform liegt das hydrogenierte Fett in der Wirkstoffsubstanz in einem Volumenanteil zwischen 20% und 80% vor und umfasst bevorzugt pflanzliche Glyceride. Ein derartiges hydrogeniertes Fett wird beispielsweise als Hartfett unter der Handelsbezeichnung Witepsol unter den Produktlinien S, E, W vertrieben. Die Auswahl des geeigneten hydrogenierten Fetts erfolgt dabei auf der Grundlage der in den Datenblättern angegebenen Schmelzpunkte und einer geeigneten Mischung der hydrogenierten Fette, falls noch eine Feinabstimmung erforderlich sein sollte.

Generell ist es bevorzugt, nur Stoffe oder Zubereitungen bei der Herstellung der Wirkstoffsubstanz zu verwenden, die in der Pharmacopöe gelistet sind und deren Einsatz für die vaginale Anwendung abgesichert ist.

Vorzugsweise liegt der Volumenanteil an psychoaktivem Tetrahydrocannabinol (THC) bei unter 0.2 Vol.-%.

Das erfindungsgemäße Verfahren zur Herstellung eines oben beschriebenen Tampons umfasst die Schritte des Bereitstellens eines Tamponkerns, der bevorzugt durch Pressen von Faserstoff zu einem Faserverbundstoff hergestellt wurde, des Herstellens der Wirkstoffsubstanz, und entweder des Beschichtens des Einführendes des Tamponkerns mit der Wirkstoffsubstanz durch Eintauchen zumindest des Einführendes in ein erwärmtes Bad mit Wirkstoffsubstanz oder Aufsprühen einer definierten Menge an Wirkstoffsubstanz, oder des Befestigens einer definierten Menge an Wirkstoffsubstanz am Einführende des Tamponkerns durch eine Klebeverbindung und/oder eine mechanische Verbindung.

Dabei ist unabhängig davon, ob die Wirkstoffsubstanz durch Eintauchen, Aufsprühen, Aufkleben oder aber durch eine mechanische Befestigung am Einführende des Tamponkerns platziert wird, darauf zu achten, dass exakt reproduzierbare Bedingungen vorliegen, damit jeweils identische Mengen an aktiver Substanz mit der darin enthaltenen chemischen Substanz appliziert werden.

Nach einer bevorzugten Ausführungsform der Erfindung erfolgt das Befestigen einer definierten Menge an Wirkstoffsubstanz am Einführende des Tamponkerns mittels mechanischer Verbindung, indem die Wirkstoffsubstanz lokal erhitzt wird und die Rückholeinrichtung durch die Wirkstoffsubstanz im lokal erhitzten Bereich hindurchgeführt wird.

Die Rückholeinrichtung ist in der Regel ein Faden aus organischer Baumwolle. Indem der Faden durch die Wirkstoffsubstanz hindurchgeführt wird, wird die Wirkstoffsubstanz am Tamponkern befestigt, solange die Wirkstoffsubstanz noch in fester Form vorliegt, d.h. bis nach dem Einführen des erfindungsgemäßen Tampons.

Allen Ausführungsformen gemeinsam ist es, dass das Einführende des Tampons durch das hydrogenierte Fett als Trägersubstanz der Wirkstoffsubstanz glatt ist, wodurch das bestimmungsgemäße Einführen des Tampons erleichtert wird.

### Kurze Beschreibung der Zeichnungen

- Figur 1: zeigt schematisch einen erfindungsgemäßen Tampon mit einer mechanischen Verbindung zwischen der Wirkstoffsubstanz und dem Tamponkern;
- Figur 2: zeigt schematisch einen erfindungsgemäßen Tampon mit einer durch Beschichten hergestellten Verbindung zwischen der Wirkstoffsubstanz und dem Tamponkern;
- Figur 3: zeigt schematisch eine Variante des erfindungsgemäßen Tampons; und
- Figur 4: zeigt schematisch eine weitere Variante des erfindungsgemäßen Tampons.

### Wege zur Ausführung der Erfindung

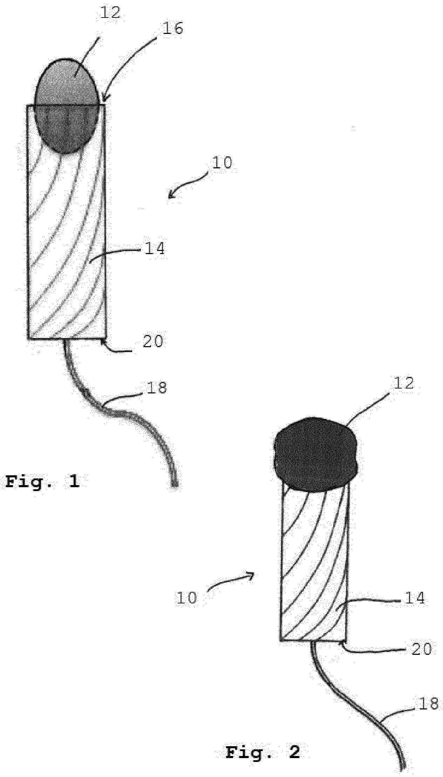

Bei der in Fig. 1 dargestellten Ausführungsform wird die Wirkstoffsubstanz 12 am Tamponkern 14 des Tampons 10 befestigt, indem die gewünschte Menge an Wirkstoffsubstanz umfassend Hartfett als Trägersubstanz, optional Milchsäure als ph-Puffersubstanz sowie die aktive Substanz, welche als chemische Substanz Cannabidiol beinhaltet, in eine ovoide Form gebracht wird. Zusätzliche kann ein Antioxidans, bevorzugt Vitamin E, in der Trägersubstanz enthalten sein.

Am Einführende 16 des Tampons wird der ovoide Körper an Wirkstoffsubstanz 12 mit dem Tamponkern 14 verbunden. Dazu wird der ovoide Körper an Wirkstoffsubstanz 12 in den Tamponkern 14 eingepresst.

Alternativ oder ergänzend kann der ovoide Körper an Wirkstoffsubstanz 12 auch mit dem Tamponkern 14 verklebt werden, wobei jegliches pharmakologisch zugelassene Haftmittel eingesetzt werden kann, das gegenüber der Wirkstoffsubstanz inert bleibt und nicht in nennenswertem Umfang in den Faserverbundstoff des Tamponkerns 14 eindringt.

Ausgehend von üblichen Abmessungen eines beispielhaften Tampons mit einem Durchmesser von 15 mm und einer Länge von 450 mm kann der ovoide Körper an Wirkstoffsubstanz 12 eine Länge von 18 mm und einen Durchmesser von 12 mm entsprechend den drei Hauptachsen mit den Abmessungen von 18mm, 12mm und 12mm besitzen. Die Wirkstoffsubstanz besitzt folglich ein Volumen von 1.36 cm³.

Eine weitere, zusätzliche Fixierung des ovoiden Körpers an Wirkstoffsubstanz 12 kann erfolgen, indem der Rückholfaden 18 durch den ovoiden Körper geführt wird. Dazu wird der ovoide Körper vor der Befestigung am Tamponkern 14 mit Hilfe von Düsen oder einem Laserstrahl lokal erhitzt und verflüssigt, so dass der Rückholfaden 18 durch die stückige, Wirkstoffsubstanz 12 durchgefädelt werden kann. Anschließend erhärtet die lokal erwärmte Wirkstoffsubstanz wieder und schließt sich fest um den Rückholfaden. Nach der Befestigung der Wirkstoffsubstanz 12 am Tamponkern 14 durch Einpressen und/oder Verkleben wird der Rückholfaden durch den Tamponkern 14 bis zum Rückholende 20 und aus diesem heraus geführt und erstreckt sich in bekannter Weise über das Rückholende 20 des Tampons 10 hinaus.

Bei der Ausführungsform nach Fig. 2 wird die Cannabidiol enthaltende Wirkstoffsubstanz 12 im Bereich des Einführendes 16 des Tampons 10 aufgebracht, indem der Tamponkern 14 entweder in erwärmte Wirkstoffsubstanz 12 eingetaucht wird oder die Wirkstoffsubstanz lokal auf den Tamponkern im Bereich des Einführendes des Tampons aufgesprüht wird.

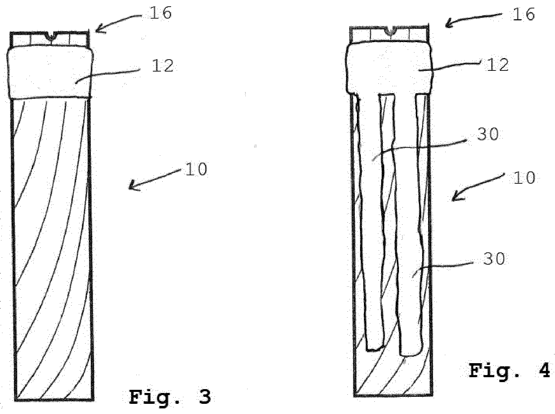

Fig. 3 und 4 zeigen weitere Alternativen der Anordnung der Wirkstoffsubstanz 12 auf dem Tamponkern. Dabei ist bei der

Ausführungsform nach Figur 3 die Wirkstoffsubstanz ringförmig im Bereich des Einführendes 16 aufgebracht, während nach der Ausführungsform nach Fig. 4 zusätzlich in Längsrichtung des Tamponkerns verlaufende Bereiche 22 mit Wirkstoffsubstanz vorgesehen sind, die den Vorteil haben, das Einführen des Tampons durch die verbesserten Gleiteigenschaften zu erleichtern. Die schematisch in Figur 4 dargestellte Anordnung der in Längsrichtung verlaufenden Bereiche 30 ist nur beispielhaft in Bezug auf die Anzahl und Länge zu verstehen.

Im Falle des Beschichtens des Einführendes 16 des Tamponkerns 14 mit Wirkstoffsubstanz 12 durch Eintauchen des Einführendes 16 in ein erwärmtes Bad mit Wirkstoffsubstanz 12 lässt sich die Dosierung und damit Menge an Wirkstoffsubstanz 12 über genau festgelegte und reproduzierbare Abläufe und Rahmenbedingungen beim Eintauchen des Einführendes 16 in das Bad mit Wirkstoffsubstanz 12 festlegen. Dabei sind die Temperaturen der erwärmten Flüssigkeit des Bads, die Eintauchzeit sowie die Eintauchtiefe, und gegebenenfalls die Anzahl von Wiederholungen zu berücksichtigen. Die Temperatur der Flüssigkeit wird in Abhängigkeit vom Schmelzpunkt eingestellt und liegt zwischen 30 °C 42 °C.

Um zu verhindern, dass der Flüssigkeit absorbierende Tamponkern 14 die Wirkstoffsubstanz 12 aufsaugt und dabei in unerwünschter Weise sein Volumen vergrößert, ist darauf zu achten, dass die Wirkstoffsubstanz 12 in dem erwärmten Bad eine Temperatur besitzt, bei der sie nicht dünnflüssig ist, sondern eine cremige, hochviskose Konsistenz aufweist. Dies besitzt den Vorteil, dass die Wirkstoffsubstanz 12 nicht in nennenswertem Umfang in den Tamponkern 14 eindringt, sondern im Wesentlichen auf dessen Oberfläche verbleibt und nach dem nachfolgenden Abkühlen dort aushärtet.

Wenn die Wirkstoffsubstanz 12 durch Aufsprühen auf dem Tamponkern 14 aufgebracht wird, sind ebenfalls exakt eingestellte und reproduzierbare Verfahrensabläufe erforderlich, um sicherzustellen, dass auf jeden Tamponkern dieselbe Menge an Wirkstoffsubstanz und damit auch aktiver Substanz aufgebracht wird. Dabei kommt es darauf an, die Sprühdauer und den Durchmesser der Düse oder der Düsen aufeinander abzustimmen. Während des Aufsprühens wird entweder der Tamponkern um seine Achse gedreht oder aber die Düse oder Düsen werden um den Tamponkern herumbewegt. Alternativ können mehrere Düsen vorgesehen sein, die um den Tampon herum angeordnet sind. Auch bei der Herstellung des Tampons durch Aufsprühen der Wirkstoffsubstanz muss die Temperatur der Wirkstoffsubstanz so weit erhöht werden, dass diese über dem Schmelzpunkt liegt und daher mittels einer Pumpe gefördert und durch die Düse oder Düsen ausgegeben werden kann.

Beiden Ausführungsformen gemeinsam sind die Alternativen zur Herstellung der Wirkstoffsubstanz. Abhängig davon, in welcher Form die aktive Substanz in der Wirkstoffsubstanz enthalten ist, liegt jeweils dieselbe vorbestimmte, wirksame Menge an Cannabidiol vor. Falls das Cannabidiol als Isolat verwendet wird, ist der Volumenanteil der aktiven Substanz in der Wirkstoffsubstanz geringer, beispielsweise zwischen 20% und 80%, während im Fall von Öl oder einer Paste, in der sich die aktive Substanz umfassend mindestens eine chemische Substanz aus der Gruppe der Cannabinoide befindet, der Volumenanteil des Öls oder der Paste in der Wirkstoffsubstanz bei bis zu 95% liegen kann. Auf diese Weise wird die Einzeldosis an Cannabinoid und insbesondere Cannabidiol pro Tampon auf einen gewünschten Zielwert eingestellt. Bevorzugt ist es allerdings, dass neben Cannabidiol noch andere chemische Stoffe aus der Gruppe der Cannabinoide in der aktiven Substanz enthalten sind, da auch andere chemische Stoffe aus der Gruppe der Cannabinoide positive Wirkungen entfalten und zudem einige chemische Stoffe aus der Gruppe der Cannabinoide auch synergetisch zusammenwirken.

Entsprechend variiert auch der Volumenanteil an hydrogeniertem Fett in der Wirkstoffsubstanz und liegt zwischen 5 % und 93 %, und kann sogar bis zu 95% betragen. Der obere Wert ist nur relevant, falls das Cannabidiol als Isolat eingesetzt wird und die aktive Substanz lediglich einen Volumenanteil von nur etwa 5% in der Wirkstoffsubstanz besitzt. Als hydrogeniertes Fett hat sich der Einsatz von Hartfett unter der Handelsbezeichnung Witepsol unter den Produktlinien S, E oder W als geeignet erwiesen. Die unter der Handelsbezeichnung Witepsol vertriebenen Hartfette eignen sich deshalb besonders für die Herstellung der Wirkstoffsubstanz, weil sie weiß oder beinahe weiß sowie geruchslos sind. Darüber hinaus bestehen diese Hartfette im Wesentlichen aus Glyceriden pflanzlichen Ursprungs. Da keine Additive zugesetzt sind, können diese Hartfette in pharmazeutischen Anwendungen eingesetzt werden.

Die Auswahl des geeigneten hydrogenierten Fetts erfolgt dabei auf der Grundlage des gewünschten Schmelzpunktes der Wirkstoffsubstanz, der zwischen 30°C und 38°C und besonders bevorzugt zwischen 34°C bis 36°C liegen sollte und entsprechend eingestellt werden kann.

Wenn Faserverlust beim Einführen und bei der Verwendung des Tampons verringert werden soll, können neben der Applikation im Bereich des Einführendes zusätzlich auch der Mittelanschnitt und/oder das Rückholende des Tamponkerns mit der Wirkstoffsubstanz oder einer weiteren Beschichtung versehen sein, beispielsweise aus gehärtetem Collagenschaum oder Gelatineschaum mit pflegenden Wirkstoffen.

Als Faserverbundstoff wird bevorzugt organischer Baumwollfaserstoff eingesetzt.

Als die optionale, den pH-Wert beeinflussende Substanz in einem Volumenanteil von etwa 2 % der Wirkstoffsubstanz wird Milchsäure verwendet, welche die Wirkstoffsubstanz stabilisiert und gleichzeitig bei der Anwendung des Tampons pflegend und regulierend wirkt.

Aus der großen Gruppe von Cannabinoiden mit über 100 unterschiedlichen Stoffen befinden sich vor allem folgende zusätzliche Cannabinoide zusätzlich zu Cannabidiol (CBD) in einer Wirkstoffsubstanz mit einer aktiven Substanz, die durch Extraktion oder Kaltpressen hergestellt wurde:
- Cannabigerolsäure (CBGA)
- Cannabigerolsäure Monomethylether (CBGAM)
- Cannabigerol (CBG)
- Cannabigerol Monometylether (CBGM)
- Cannabigerovarinsäure (CBGVA)
- Cannabigerovarin (CBGV)
- Cannabichromensäure (CBCA)
- Cannabichromen (CBC)
- Cannabichromevarinsäure (CBCVA)
- Cannabichromevarin (CBCV)
- Cannabidiolsäure (CBDA)
- Cannabidiol Monomethylether (CBDM)
- Cannabidiol C₄ (CBD-C₄)
- Cannabidivarinsäure (CDBVA)
- Cannabidivarin (CBDV)
- Cannabidiorcol (CDB-C₁)
- Delta-9-Tetrahydrocannabinolsäure A (THCA-A)
- Delta-9-Tetrahydrocannabinolsäure B (THCA-B)
- Delta-9-Tetrahydrocannabinol (THC)
- Delta-9-Tetrahydrocannabinolsäure C₄ (THCA-C₄)
- Delta-9-Tetrahydrocannabinol C₄ (THC-C₄)
- Delta-9-Tetrahydrocannabivarinsäure (THCVA)
- Delta-9-Tetrahydrocannabivarin (THCV)
- Delta-9-Tetrahydrocannabiorcolsäure (THCA-C₁)
- Delta-9-Tetrahydrocannabiorcol (THC-C₁)
- Delta-7-cis-Isotetrahydrocannabivarin
- Delta-8-Tetrahydrocannaninolsäure (Δ⁸-THCA)
- Delta-8-Tetrahydrocannaninol (Δ⁸-THC)
- Cannabicyclolsäure (CBLA-C₅)
- Cannabicyclol (CBL-C₅)
- Cannabicyclovarin (CBLV)
- Cannabinol Methylether (CBNM)
- Cannabinol-C₄ (CBN-C₄)
- Cannabivarin (CBV)
- Cannabinol-C₂ (CBN-C₂)
- Cannabiorcol (CBN-C₁)
- Cannabinodiol (CBND)
- Cannabinodivarin (CBVD)
- Cannabitriol (CBT)
- 10-Ethoxy-9-hydroxy-delta-6°-Tetrahydrocannabinol
- 8,9-Dihydroxy-delta-6°-Tetrahydrocannabinol
- Cannabitriolvarin (CBTV)
- Ethoxy-Cannabitriolvarin (CBTVE)
- Dehydrocannabifuran (DCBF)
- Cannabifuran (CBF)
- Cannabichromanon (CBCN)
- Cannabicitran (CBT)
- 10-Oxo-delta-6a-Tetrahydrocannabinol (OTHC)
- Delta-9-cis-Tetrahydrcannabinol (cis-THC)
- Cannabiglendol (OH-iso-HHCV)
- Cannabiripsol (CBR)
- Trihydroxy-delta-9-Tetrahydrocannabinol (triOH-THC).

Die wichtigsten Stoffe, die aus Cannabis gewonnen werden, sind dabei aus der obigen Auflistung die Stoffe CBG, THC, CBD, CBC, CBGV, THCV, CBDV und CBCV.

Dabei können einige Cannabinoide neben Cannabidiol (CBD) erwünschte Begleitsubstanzen sein. Cannabigerol (CBG), Cannabigerolsäure (CBGA), Cannabichromen (CBC) und Cannabidiolsäure (CBDA) beispielsweise besitzen antibiotische Wirkung, während Cannabigerol und Delta-9-Tetrahydrocannabinol (THC) darüber hinaus auch eine schmerzlindernde und entzündungshemmende Wirkung entfalten. Auch die anti-mykotischen Eigenschaften von Cannabigerol können einen positiven Zusatzeffekt haben.

### Beispiel

### Produktionsbeschreibung für ein CBD Tampon mit 100mg aktivem CBD Wirkstoff

Es werden ca. 10 Gramm Witepsol E76 Pastillen zusammen mit ca. 5 Gramm CBD Paste (50% CBD Gehalt) sowie ca. 0.3 Gramm Milchsäure (Lacidum Lacticum) in ein Glasgefäß gegeben, langsam erhitzt und dabei gerührt, bis eine gleichmäßige cremeartige Emulsion entsteht. Bei Erreichen einer Temperatur im Bereich zwischen 30°C und 40°C hat die Wirkstoffsubstanz eine Viskosität, die eine ideale Verarbeitung gewährleisten kann.

Auf die jeweilige Tamponspitze sollen 612mg der Wirkstoffsubstanz aufgetragen werden. Die Wirkstoffsubstanz setzt sich aus 400mg Witepsol E76 als Trägersubstanz (65%), 200mg CBD Paste (33%) sowie 12mg Milchsäure (2%) zusammen. Zwei unterschiedliche Applikationsverfahren wurden untersucht:
1) Applikation der Wirkstoffsubstanz mit einer Düse
   Die Wirkstoffsubstanz wird anschließend mit einer dünnen Düse gleichmäßig um die Tamponspitze aufgetragen. Hierbei ist der Durchmesser der Düse sowie die Temperatur der Substanz so zu wählen, dass die Wirkstoffsubstanz durch den Tamponkern nicht aufgesaugt wird. Vielmehr soll sich die Wirkstoffsubstanz gleichmäßig auf der Oberfläche des Tamponkerns im Bereich des Einführendes (Tamponspitze) verteilen.
2) Applikation der Wirkstoffsubstanz mittels Immersionsverfahren / Tauchverfahren
   Bei diesem Verfahren wird die Tamponspitze kurz in ein Becken eingetaucht, das die Wirkstoffsubstanz enthält. Es ist darauf zu achten, dass die Viskosität der Wirkstoffsubstanz hoch genug ist, so dass die Wirkstoffsubstanz cremeartig ist. Dies hat den Vorteil, dass ein starkes Aufsaugen durch den Tamponkern verhindert wird. Um die applizierte Wirkstoffsubstanz zu dosieren, kann der Tamponkern zum schnelleren Aushärten der Wirkstoffsubstanz auf dem Tamponkern heruntergekühlt werden. Weiterhin spielt die generelle Verweildauer beim Eintauchen des Tamponkerns in das Wirkstoffbad eine Rolle. Auch kann eine Dosierung durch mehrfaches "Intervallartiges Eintauchen" erzielt werden. Bei Zimmertemperatur des Tamponkerns und einer auf 30°C bis 40°C erwärmten Wirkstoffsubstanz hat sich ein 3-faches bis 5-faches Eintauchen mit einer jeweiligen Verweildauer von 1 bis 2 Sekunden als geeignet gezeigt.

## Patentansprüche

1. Tampon zur vaginalen Anwendung für medizinische oder hygienische Zwecke mit einem Einführende (16), einem Mittelabschnitt und einem Rückholende (20), umfassend:
- einen Flüssigkeit absorbierenden Tamponkern (14);
- eine Rückholeinrichtung, vorzugsweise einen Rückholfaden (18), die sich vom Rückholende (20) über dieses hinaus erstreckt;
- optional eine bereichsweise auf dem Tamponkern (14) aufgebrachte, äußere Gleitmittelbeschichtung; sowie
- eine Wirkstoffsubstanz (12) zumindest im Bereich des Einführendes (16), welche im Bereich des Einführendes (16) als Beschichtung oder als stückiges Material vorliegt, das mit dem Tamponkern (14) verbunden ist, wobei
- die Wirkstoffsubstanz umfasst:
- eine aktive Substanz umfassend mindestens eine chemische Substanz aus der Gruppe der Cannabinoide; und
- ein hydrogeniertes Fett als Trägersubstanz
**dadurch gekennzeichnet, dass**
- der Tamponkern (14) aus einem gepressten Faserverbundstoff besteht;
- die aktive Substanz umfassend mindestens eine chemische Substanz aus der Gruppe der Cannabinoide zu mindestens 90 Vol.-% Cannabidiol umfasst, das als Isolat oder Destillat vorliegt; und
- die aktive Substanz in einem Volumenanteil von 20% bis 80% der Wirkstoffsusbtanz (12) vorliegt.

2. Tampon nach Anspruch 1, **dadurch gekennzeichnet, dass** der Faserverbundstoff organischer Baumwollfaserstoff ist.

3. Tampon nach Anspruch 1 oder 2, weiter umfassend:
- eine den ph-Wert beeinflussende Substanz in einem Volumenanteil von etwa 2% der Wirkstoffsubstanz (12); wobei
- die den ph-Wert beeinflussende Substanz vorzugsweise eine ph-Puffersubstanz ist, die Milchsäure umfasst.

4. Tampon nach einem der vorhergehenden Ansprüche, **dadurch gekennzeichnet, dass** das hydrogenierte Fett in der Wirkstoffsubstanz (12) in einem Volumenanteil zwischen 20% und 80% vorliegt und bevorzugt pflanzliche Gyceride umfasst.

5. Tampon nach einem der vorhergehenden Ansprüche, **dadurch gekennzeichnet, dass** die Wirkstoffsubstanz (12) stückig vorliegt und so mit dem Tamponkern (14) verbunden ist, dass die Wirkstoffsubstanz (12) das Einführende (16) des Tampons (10) darstellt.

6. Verfahren zur Herstellung eines Tampons (10) nach einem der vorhergehenden Ansprüche, umfassend die Schritte:
(a) Bereitstellen eines Tamponkerns (14);
(b) Herstellen der Wirkstoffsubstanz (12); sowie entweder
(c1) Beschichten zumindest des Einführendes (16) des Tamponkerns (14) mit der Wirkstoffsubstanz (12) durch Eintauchen des Einführendes (16) in ein erwärmtes Bad mit Wirkstoffsubstanz (12) oder Aufsprühen einer definierten Menge an Wirkstoffsubstanz (12); oder
(c2) Befestigen einer definierten Menge an Wirkstoffsubstanz (12) am Einführende (16) des Tamponkerns (14) durch eine Klebeverbindung und/oder eine mechanische Verbindung.

7. Verfahren nach Anspruch 6, wobei im Schritt (c2) die Wirkstoffsubstanz (12) lokal erhitzt wird, und die Rückholeinrichtung (18) durch die Wirkstoffsubstanz (12) im lokal erhitzten Bereich hindurchgeführt wird.

## Claims

1. A tampon for vaginal use for medical or hygienic purposes with an insertion end (16), a middle section and a retrieval end (20), comprising:
- a liquid-absorbing tampon core (14);
- a retrieval apparatus, preferably a retrieval thread (18), which extends from the retrieval end (20) beyond it;
- optionally, an external lubricant coating, applied to the tampon core (14) in certain areas; and
- an active drug substance (12) at least in the area of the insertion end (16), which is present in the area of the insertion end (16) as coating or as lumpy material, that is connected to the tampon core (14), wherein
- the active drug substance comprises:
- an active substance, comprising at least one chemical substance from the group of cannabinoids; and
- a hydrogenated fat as a carrier substance,
**characterized in that**
- the tampon core (14) consists of a pressed fibre composite material;
- the active substance, comprising at least one chemical substance from the group of cannabinoids, comprises at least 90 vol % cannabidiol, which is present as an isolate or distillate; and
- the active substance is present in a volume fraction of 20% to 80% of the active drug substance (12).

2. The tampon according to claim 1, **characterized in that** the fibre composite material is organic cotton fibre.

3. The tampon according to claim 1 or 2, further comprising:
- a pH-value-influencing substance in a volume fraction of approximately 2% of the active drug substance (12); wherein
- the pH-value-influencing substance is preferably a pH-buffer substance, which comprises lactic acid.

4. The tampon according to any one of the preceding claims, **characterized in that** the hydrogenated fat in the active drug substance (12) is present in a volume fraction between 20% and 80% and preferably comprises vegetable glycerides.

5. The tampon according to any one of the preceding claims, **characterized in that** the active drug substance (12) is in a lumpy form and is connected to the tampon core (14) in such a way that the active drug substance (12) forms the insertion end (16) of the tampon (10).

6. A method for manufacturing a tampon (10) according to any one of the preceding claims, comprising the steps:
(a) providing a tampon core (14);
(b) manufacturing the active drug substance (12); and either
(c1) coating at least the insertion end (16) of the tampon core (14) with the active drug substance (12) by immersing the insertion end (16) in a heated bath, containing the active drug substance (12), or by spraying on a defined quantity of the active drug substance (12); or
(c2) attaching a defined amount of active drug substance (12) to the insertion end (16) of the tampon core (14) by means of an adhesive bond and/or a mechanical bond.

7. The method according to claim 6, wherein, in step (c2), the active drug substance (12) is locally heated, and the retrieval apparatus (18) is passed through the active drug substance (12) in the locally heated area.

## Revendications

1. Tampon pour application vaginale à des fins médicales ou hygiéniques comportant une extrémité d'insertion (16), une section centrale et une extrémité de retrait (20), comprenant :
- un cœur (14) de tampon absorbant les liquides ;
- un dispositif de retrait, de préférence un fil de retrait (18), qui s'étend depuis l'extrémité de retrait (20) au-delà de celle-ci ;
- facultativement un revêtement extérieur lubrifiant appliqué par endroits sur le cœur de tampon (14) ; ainsi que - un agent actif (12) au moins dans la zone de l'extrémité d'insertion (16), qui est présent dans la zone de l'extrémité d'insertion (16) en tant que revêtement ou en tant que matériau en morceaux relié au cœur de tampon (14), dans lequel
- l'agent actif comprend :
- une substance active comprenant au moins une substance chimique du groupe des cannabinoïdes ; et
- une graisse hydrogénée comme substance porteuse **caractérisé en ce que**
- le cœur de tampon (14) est constitué d'un matériau composite de fibres comprimées ;
- comprend la substance active comprenant au moins une substance chimique du groupe des cannabinoïdes à au moins 90 % vol de cannabidiol, qui se présente sous forme d'isolat ou de distillat ; et
- la substance active est présente dans une proportion volumique de 20 % à 80 % de l'agent actif (12).

2. Tampon selon la revendication 1, **caractérisé en ce que** le matériau composite de fibres est un tissu de fibres de coton organique.

3. Tampon selon la revendication 1 ou la revendication 2, comprenant en outre :
- une substance influençant le pH dans une proportion en volume d'environ 2 % de l'agent actif (12) ; dans lequel
- la substance influençant le pH est de préférence une substance tampon de pH comprenant de l'acide lactique.

4. Tampon selon l'une quelconque des revendications précédentes, **caractérisé en ce que** la graisse hydrogénée dans l'agent actif (12) est présente dans une proportion en volume comprise entre 20 % et 80 % et comprend préférablement des glycérides végétaux.

5. Tampon selon l'une quelconque des revendications précédentes, **caractérisé en ce que** l'agent actif (12) est présent en morceaux et est relié au cœur de tampon (14) de sorte que l'agent actif (12) représente l'extrémité d'insertion (16) du tampon (10).

6. Procédé de fabrication d'un tampon (10) selon l'une quelconque des revendications précédentes, comprenant les étapes :
(a) mise à disposition d'un cœur de tampon (14) ;
(b) la fabrication de l'agent actif (12) ; et soit
(c1) le revêtement d'au moins l'extrémité d'insertion (16) du cœur de tampon (14) avec l'agent actif (12) par immersion de l'extrémité d'insertion (16) dans un bain chauffé d'agent actif (12) ou par pulvérisation d'une quantité définie d'agent actif (12) ; soit
(c2) la fixation d'une quantité définie d'agent actif (12) à l'extrémité d'insertion (16) du cœur de tampon (14) par un composé adhésif et/ou un composé mécanique.

7. Procédé selon la revendication 6, dans lequel, à l'étape (c2), l'agent actif (12) est chauffé localement et le dispositif de retrait (18) est traversé par l'agent actif (12) dans la région chauffée localement.
